Europäisches Patentamt

European Patent Office

Office européen des brevets

(11) Publication number: **0 132 352**
A2

# (12) EUROPEAN PATENT APPLICATION

(21) Application number: **84304805.9**

(22) Date of filing: **13.07.84**

(51) Int. Cl.⁴: **B 65 D 83/14**, A 61 M 11/00

(30) Priority: **15.07.83 GB 8319150**

(43) Date of publication of application: **30.01.85**
Bulletin 85/5

(84) Designated Contracting States: **AT BE CH DE FR GB IT LI LU NL SE**

(71) Applicant: **GLAXO GROUP LIMITED, Clarges House 6-12 Clarges Street, London W1Y 8DH (GB)**

(72) Inventor: **Rand, Paul Kenneth, 29 Kipling Close, Hitchin Herts (GB)**
Inventor: **Hallworth, Gerald Wynn, 8 Warner Road, Ware Herts (GB)**
Inventor: **Price, Philip Thomas, 4 Brantwood Avenue, Isleworth, Middlesex (GB)**

(74) Representative: **Fentiman, Denis Richard et al, Elkington and Fife High Holborn House 52/54 High Holborn, London WC1V 6SH (GB)**

(54) **Aerosol applicator device.**

(57) An applicator for dispensing medicaments from a pressurised dispensing container (7), that is to say an aerosol, has a body (1) which is adapted to receive the aerosol dispensing container (7). An outlet spout (2) leads outwards from the body (1). An outlet member (17) is located in the spout (2). The said outlet member (17) has a passage (8) arranged at one end to receive contents discharged from the aerosol dispensing container and terminating at the other end in an outlet (18) directed towards the outlet end of the spout (2).

Title:        Aerosol Applicator Device

This invention relates to applicators for dispensing medicaments from a pressurised dispensing container, i.e. a so-called aerosol.   Aerosols containing a medicament and a propellant and are commonly used, for example, in the treatment of conditions of the nose, throat or mouth. The medicament and propellant are applied to the affected parts in the form of spray by means of nasal or oral applicators or dispensers containing the aerosols.   The specification of UK Patent No. 1402638 describes a particularly successful known applicator.   The applicator described in that specification comprises a body which is open at one end and which is arranged to receive and retain a pressurised aerosol dispensing container.   An outlet spout for a mixture of medicament dispensed from the container and air leads laterally out of the body. A cover is hingedly connected to the body and is arranged to enclose the body and at the same time to enclose the outlet spout.

An object of the present invention is to provide an improved aerosol applicator device of this character. According to the invention, an aerosol applicator device comprises an applicator for dispensing medicaments from a pressurised dispensing container, the said applicator comprising a body which is adapted to receive an aerosol dispensing container, an outlet spout leading outwards from the body, and an outlet member located in the spout, the said outlet member having a passage arranged at one end so as to receive contents discharged from the aerosol dispensing container and terminating at the other end in an outlet directed towards the outlet end of the spout. The outlet member may be arranged to fit on a nozzle of a stemblock positioned in the body of the applicator device to receive a valve stem of the container.   The outlet member alternatively may have an inlet passage arranged to receive a valve stem of the container. The outlet member has a passage which may be a capillary passage leading to the outlet, the said outlet being located at or near the outlet end of the spout. The outlet may be internally bevelled or other means of diffusing the contents of the container passing through the outlet may be provided. The external surface of the insert may be fluted, finned or ribbed, the spaces between at least some of the flutes, fins or ribs providing air passages through which a patient may inhale air. A locating fin may be arranged inside the spout or the body so as to engage in a space between two adjacent flutes or ribs and thereby to locate the insert in position. The outlet member may be made of metal or it may be made of a plastics material.

Three embodiments of the invention are illustrated in the accompanying drawings in which:

Figure 1 is a sectional view of one embodiment of an applicator device according to the invention,

Figure 2 is a front view partly in section of the same device,

Figure 3 is a perspective view of an insert forming part of the device,

Figure 4 is a front view of the insert located in a spout of the device illustrated in Figure 1,

Figure 5 is a plan view, partly in section of the device,

Figure 6 is a sectional detail view illustrating a preferred embodiment,

Figure 7 is a perspective view of an outlet member of the same embodiment,

Figure 8 is a sectional view, and

Figure 9 is a perspective view of a modification of the embodiment illustrated in Figures 6 and 7.

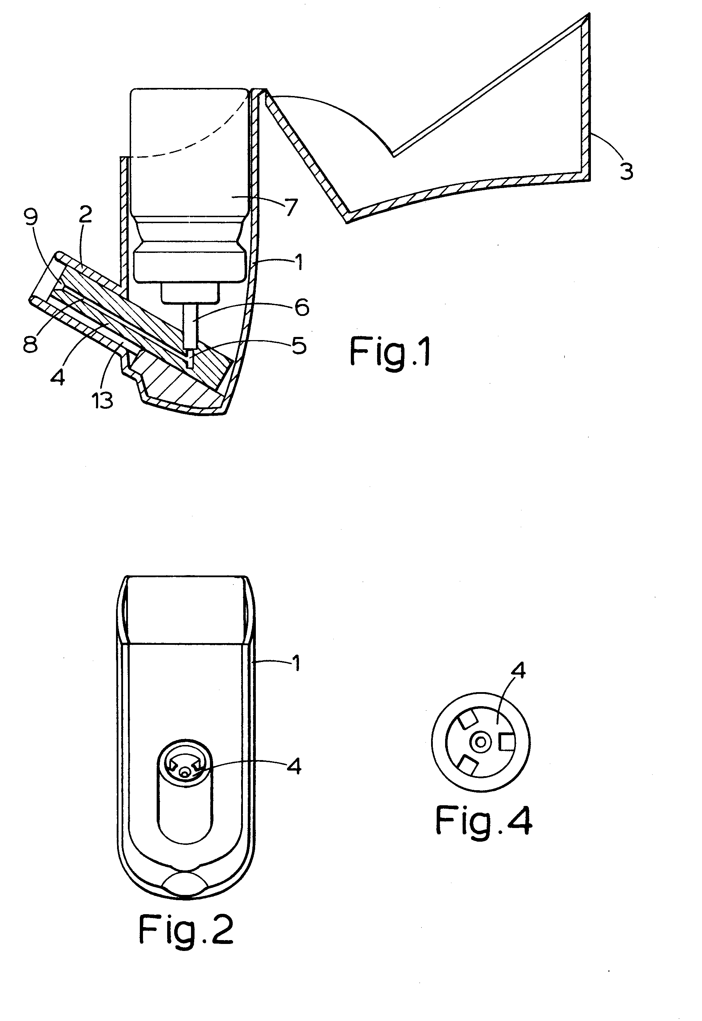

In the embodiment of the invention illustrated in Figures 1 to 5, an applicator device for dispensing medicaments from an aerosol container comprises a body 1 with an outlet spout 2 leading from a lower end of the body. A cover 3 is hingedly connected to the body 1 and when positioned in its closed position it will close the body 1 as well as enclosing the outlet spout 2. Such an arrangement is generally described in the specification of U.K. Patent No. 1402638.

In the improved construction provided by this embodiment of the present invention, outlet member 4 which in this embodiment is an insert, is a sliding fit inside the spout 2 and the lower part of the body 1. The member 4 may be made of metal or of a plastics material. The member 4 has an inlet passage 5 positioned so as to receive a valve stem 6 of a pressurised aerosol container 7. Thus, when the container 7 is depressed, the valve opens and a metered quantity of the contents of the container 7 can pass through the valve stem into the inlet passage 5.

A capillary outlet passage 8 extends from the inlet passage 5 to an atomiser nozzle or outlet 9 at the outlet end of the insert. The member 4 is bevelled at the outer end of the passage 8 to provide the atomiser nozzle 9. The nozzle 9 is positioned near the outlet opening of the spout 2. The external surface of the member 4 is preferably fluted, with spaces 10, 11 and 12 between the flutes. Two of the spaces 11, 12 serve as passages through which air may be inhaled by a patient using the device. The third space 10 is arranged to receive a locating fin 13 at the end of the spout remote from the outlet opening. This fin serves to locate the member 4 in its correct position when it is pushed as far as possible into the spout and body as illustrated in the drawing.

- 4 -

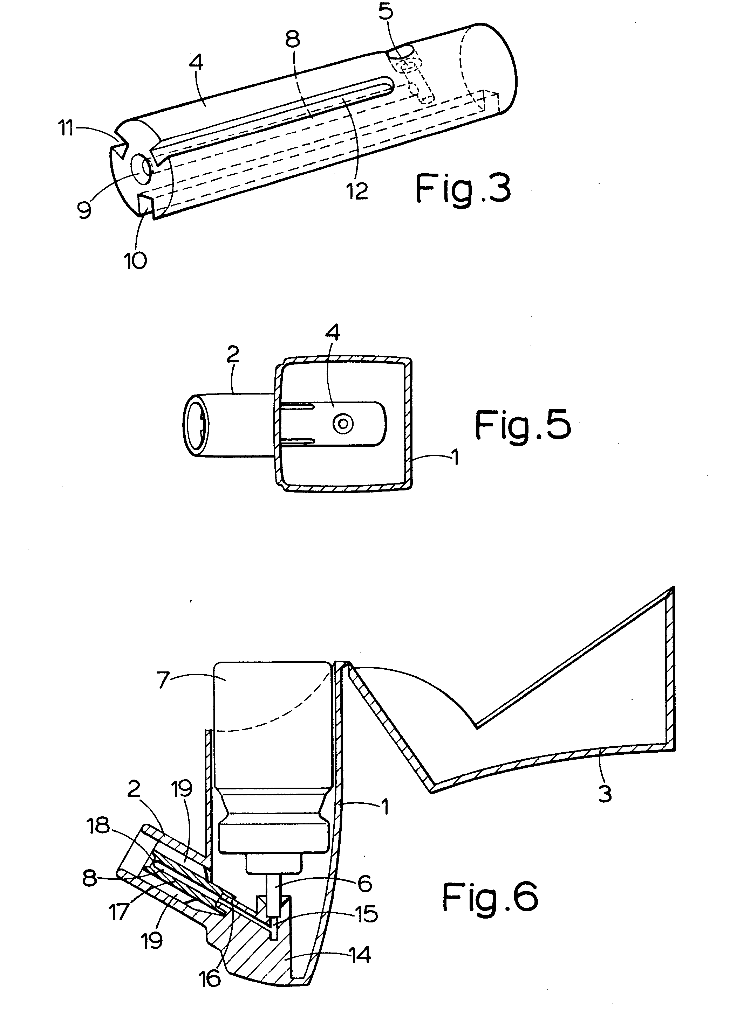

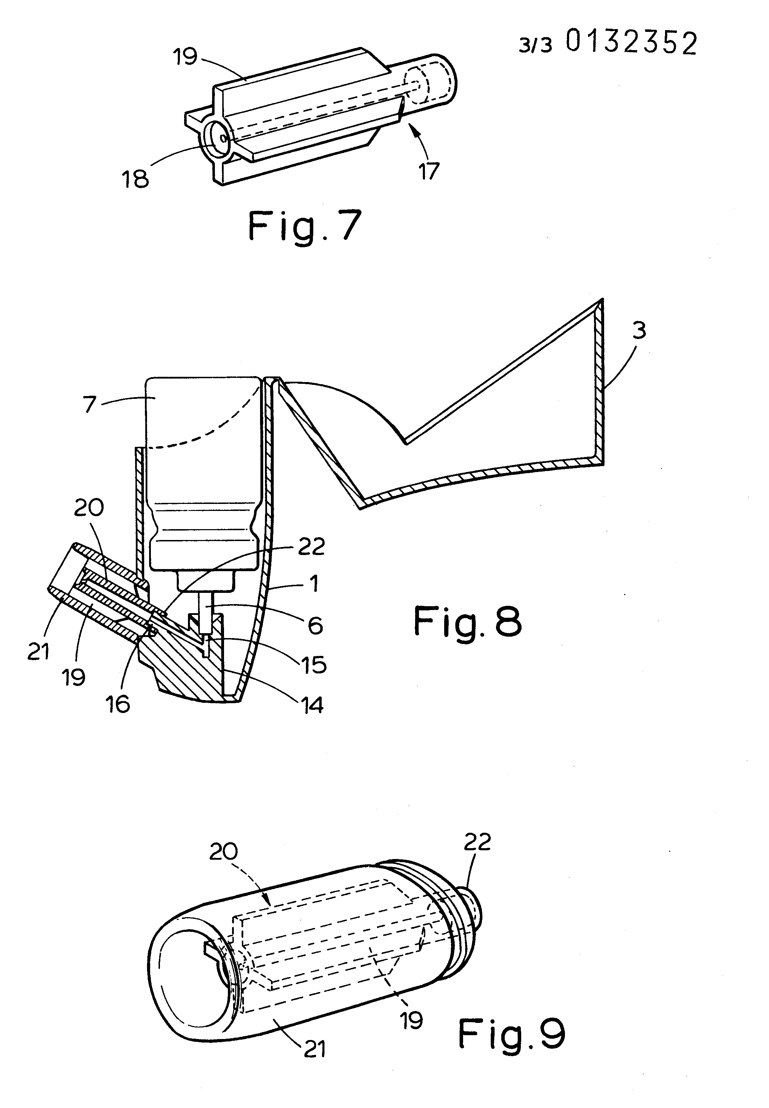

A more preferable embodiment is illustrated in Figures 6 and 7 in which like parts are designated by like reference numerals. In this embodiment, a stemblock 14 is located in the body 1 of the applicator device near its junction with the spout 2. The stemblock 14 has an inlet passage 15 arranged to receive the valve stem 6 of the aerosol container 7, and an outlet or male connector 16 communicating with the passage 15, and directed towards the spout 2. This arrangement is generally similar to that described in U.K. Patent No. 1402638. An outlet member 17 is arranged in the spout 2. The outlet member has a female recess at one end of the member in which the male outlet 16 is fitted. The capillary outlet passage 8 leads to the other end of the member 17 terminating in an outlet 18 near the outlet end of the spout 2. A plurality of radial ribs or fins 19 are provided on the member 17, the edges of the ribs or fins being a fit with the inner surface of the spout 2 so that the member 17 is located in position.

A further modification is illustrated in Figures 8 and 9 in which like parts are again designated by like reference numerals. In this embodiment, an outlet member 20 of generally similar construction to the one illustrated in Figures 6 and 7 is preformed as a unit with an outlet spout 21. A female extension 22 of the outlet member 20 is engaged with the male outlet 16 of the stemblock 14.

CLAIMS:

1.      An applicator for dispensing medicaments from a
pressurised dispensing container, the said applicator
comprising a body which is adapted to receive an aerosol
dispensing container, an outlet spout leading outwards
from the body, and an outlet member located in the spout,
the said outlet member having a passage arranged at one
end so as to receive contents discharged from the aerosol
dispensing container and terminating at the other end
in an outlet directed towards the outlet end of the spout.

2.      An applicator as claimed in claim 1, comprising a
stemblock located in the body near its junction with
the outlet spout, the said stemblock having an inlet
passage arranged to receive a valve stem of an aerosol
container and a male connector and outlet directed towards
the spout and wherein the outlet member has a female
recess communicating with an outlet passage in the outlet
member and engaged with the male connector and outlet.

3.      An applicator as claimed in claim 2 wherein the
outlet member has a plurality of radial ribs which fit
with the inner surface of the spout.

4.      An applicator as claimed in either one of claims
2 or 3 wherein the outlet member and the spout are preformed as a unit and then secured to the body.

5.      An applicator as claimed in claim 1, wherein the
outlet member extends through the spout into the body
and has an inlet passage arranged to receive a valve
stem of an aerosol container, the said inlet passage
communicating with an outlet passage extending through
the outlet member.

6.    An applicator according to any one of the preceding claims wherein the outlet member has a capillary outlet passage which is internally bevelled at an outlet end to provide an atomiser nozzle.

7.    An applicator for dispensing medicaments from a pressurised dispensing container substantially as described with reference to Figures 1 - 5 or to Figures 6 and 7 or to Figures 8 and 9 of the accompanying drawings.

Fig.1

Fig.2

Fig.4

0132352

Fig.3

Fig.5

Fig.6

Fig. 7

Fig. 8

Fig. 9